(19) Europäisches Patentamt
European Patent Office
Office européen des brevets

(11) Veröffentlichungsnummer: **0 564 402 B1**

(12) **EUROPÄISCHE PATENTSCHRIFT**

(45) Veröffentlichungstag der Patentschrift: **07.06.95**

(51) Int. Cl.6: **C07C 43/32**, C07C 233/17, C11D 1/72, B01F 17/00, C08G 65/32

(21) Anmeldenummer: **93810201.9**

(22) Anmeldetag: **22.03.93**

# (54) Schaumarme oberflächenaktive Verbindungen.

(30) Priorität: **01.04.92 CH 1051/92**

(43) Veröffentlichungstag der Anmeldung:
**06.10.93 Patentblatt 93/40**

(45) Bekanntmachung des Hinweises auf die Patenterteilung:
**07.06.95 Patentblatt 95/23**

(84) Benannte Vertragsstaaten:
**BE DE ES FR GB IT NL**

(56) Entgegenhaltungen:
**EP-A- 0 039 859**
**EP-A- 0 040 713**
**EP-A- 0 054 366**
**EP-A- 0 093 791**
**DE-A- 2 062 034**
**FR-A- 2 204 683**
**FR-A- 2 370 788**
**US-A- 2 867 667**

(73) Patentinhaber: **CIBA-GEIGY AG**
**Klybeckstrasse 141**
**CH-4002 Basel (CH)**

(72) Erfinder: **Haase, Jürg**
**Bettingerstrasse 265**
**CH-4125 Riehen (CH)**
Erfinder: **Guth, Christian**
**Rheinstrasse 25**
**CH-4127 Birsfelden (CH)**
Erfinder: **Stehlin, Albert**
**Rue de Bougue 6**
**F-68300 Rosenau (FR)**

EP 0 564 402 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft schaumarme, nichtionische, oberflächenaktive Verbindungen, die mit Orthoestergruppen endverschlossen sind, sowie deren Herstellung.

Nichtionische oberflächenaktive Verbindungen weisen in vielen technischen Anwendungen oft ein zu hohes Schaumvermögen auf. Dieser Nachteil kann, durch eine Einführung von hydrophoben Endgruppen in die oberflächenaktive Verbindung, behoben werden. Zu diesem Zweck wurden bereits terminale OH-Gruppen solcher nichtionischer oberflächenaktiver Verbindungen mit Alkylisocyanaten, Oxypropylen, Alkylchlorid oder Benzylchlorid verschlossen.

US-A-2 867 667 und DE-A-2 062 034 beschreiben alkoxylierte Alkohole, die mit Orthoestergruppen endverschlossen sind und die Verwendung dieser Verbindung z.B. als Lösungsmittel oder in hydraulischen Flüssigkeiten. In EP-A-93791, FR-A-2 370 788 und FR-A-2 204 683 werden schaumarme, oberflächenaktive, mit Acetalen oder Kohlensäureestern endverschlossene, alkoxylierte Alkohole und ihre Verwendung als nonionische Tenside offenbart.

Es wurde nun gefunden, dass oberflächenaktive Verbindungen, deren terminale OH-Gruppen mit Orthoestergruppen verschlossen sind, eine für alkalische oder neutrale Anwendungen ausreichende Stabilität aufweisen und zudem ein gutes Netzverhalten, sowie eine hohe Schaumarmut zeigen.

Die erfindungsgemässen Verbindungen entsprechen der Formel

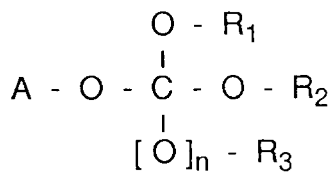 (1)

worin

- $R_1$ und $R_2$ unabhängig voneinander unsubstituiertes oder substituiertes $C_1$-$C_{10}$-Alkyl, $C_2$-$C_{10}$-Alkenyl oder $R_1$ gleich A;
- n 0 oder 1;
- $R_3$ Wasserstoff, unsubstituiertes oder substituiertes $C_1$-$C_{10}$-Alkyl, oder $C_2$-$C_{10}$-Alkenyl; oder
- $R_1$ und $R_2$ gemeinsam eine geradkettige oder verzweigte $C_2$-$C_5$-Alkylenbrücke; und

die Gesamtzahl der C-Atome in $R_1$, $R_2$ und $R_3$ zusammen nicht mehr als 10 beträgt, wobei die C-Atome für $R_1$ gleich A nicht berücksichtigt werden;

- A den Rest $R_4$-X-($C_2$-$C_4$-Alkyl-O-$)_m$- $C_2$-$C_4$-Alkyl-;
- $R_4$ unsubstituiertes oder substituiertes $C_8$-$C_{26}$-Alkyl oder $C_8$-$C_{26}$-Alkenyl; im Phenylrest substituiertes Phenyl, Biphenyl oder Phenyl-$C_1$-$C_{13}$-Alkyl;
- X

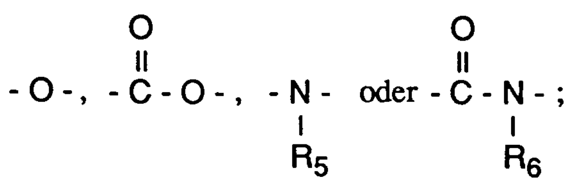

oder ;

- $R_5$ und $R_6$ unabhängig voneinander Wasserstoff, unsubstituiertes oder substituiertes $C_1$-$C_8$-Alkyl, -($C_2$-$C_4$-Alkyl-O-$)_o$- $C_2$-$C_4$-Alkyl-OH, oder

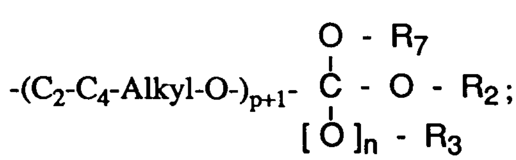

- $R_7$ unsubstituiertes oder substituiertes $C_1$-$C_{10}$-Alkyl oder $C_2$-$C_{10}$-Alkenyl; und

m, o und p unabhängig voneinander 0 bis 100 bedeutet.

Bevorzugt sind Verbindungen, worin

$R_1$ und $R_2$ unabhängig voneinander unsubstituiertes oder substituiertes $C_1$-$C_{10}$-Alkyl; oder $C_2$-$C_{10}$-Alkenyl;

n 0 oder 1;

$R_3$ Wasserstoff, unsubstituiertes oder substituiertes $C_1$-$C_{10}$-Alkyl, oder $C_2$-$C_{10}$-Alkenyl; oder

$R_1$ und $R_2$ gemeinsam eine geradkettige oder verzweigte $C_2$-$C_5$-Alkylenbrücke;

bedeutet und die Gesamtzahl der C-Atome in $R_1$, $R_2$ und $R_3$ zusammen nicht mehr als 10 beträgt.

Die Reste $R_1$, $R_2$, $R_3$, $R_5$, $R_6$ und $R_7$ können dabei unabhängig voneinander durch Cyano- oder $C_1$-$C_4$-Alkoxygruppen einfach oder mehrfach substituiert sein.

Der Rest $R_4$ kann im Falle eines $C_8$-$C_{26}$-Alkyls oder $C_8$-$C_{26}$-Alkenyls durch Cyano-, $C_1$-$C_4$-Alkoxygruppen einfach oder mehrfach substituiert sein. Ist der Rest $R_4$ Phenyl, Biphenyl oder Phenyl-$C_1$-$C_{13}$-Alkyl so kann dieser einfach durch $C_1C_{10}$-Alkyl, $C_1$-$C_{10}$-Alkoxy, oder Cyano; und bis zu dreimal durch $C_1$-$C_4$-Alkyl, $C_1$-$C_4$-Alkoxy oder Cyano; vorzugsweise einfach durch $C_1$-$C_{16}$-Alkyl oder $C_1$-$C_4$-Alkoxy; und besonders bevorzugt einfach durch $C_6$-$C_{12}$-Alkyl substituiert sein.

$R_4$ ist insbesondere unsubstituiertes $C_8$-$C_{18}$-Alkyl wie $CH_3$-$(CH_2)_{7-12}$- oder $C_8$-$C_{18}$-Alkenyl wie $CH_3$-$(CH_2)_7$-$CH=CH$-$(CH_2)_8$- ; oder mit Cyano oder $C_1$-$C_4$-Alkoxygruppen einfach oder mehrfach substituiertes $C_8$-$C_{18}$-Alkyl; oder $C_6$-$C_{12}$-Alkylphenyl. Besonders bevorzugt ist $R_4$ unsubstituiertes $C_8$-$C_{18}$-Alkyl wie $CH_3$-$(CH_2)_{7-12}$-; oder $C_8$-$C_{18}$-Alkenyl wie $CH_3$-$(CH_2)_7$-$CH=CH$-$(CH_2)_8$-; oder $C_6$-$C_{12}$-Alkylphenyl wie Nonylphenyl.

Als X ist -O- oder

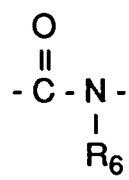

bevorzugt, wobei -O- besonders bevorzugt ist.

Als ($C_2$-$C_4$-Alkyl-O-$)_m$-, ($C_2$-$C_4$-Alkyl-O-$)_o$- und ($C_2$-$C_4$-Alkyl-O-$)_{p+1}$-Ketten kommen insbesondere solche Strukturen in Frage, die sich durch Oligomerisierung von Ethylenoxid, oder Gemischen aus Ethylenoxid, Propylenoxid, und/oder Butylenoxid herleiten. Bevorzugt sind Oligomere, der in der jeweiligen Definition genannten Kettenlänge, die sich aus -$CH_2$-$CH_2$-O-, oder Mischungen von -$CH_2$-$CH_2$-O- und -$CH(CH_3)$-$CH_2$-O- Einheiten aufbauen. Besonders bevorzugt sind Ketten die aus -$CH_2$-$CH_2$-O- Einheiten aufgebaut sind, wobei m, o und p unabhängig voneinander bevorzugt 0 bis 50 bedeutet.

Bei den Orthoestern sind solche Verbindungen bevorzugt, worin $R_1$, $R_2$ und $R_7$ unabhängig voneinander unsubstituiertes $C_1$-$C_4$-Alkyl oder $C_2$-$C_4$-Alkenyl; $R_3$ Wasserstoff oder unsubstituiertes $C_1$-$C_4$-Alkyl oder $C_2$-$C_4$-Alkenyl; oder $R_1$ und $R_2$, $R_1$ und $R_3$, $R_2$ und $R_7$ oder $R_3$ und $R_7$ gemeinsam eine $C_2$-$C_4$-Alkylenbrücke bedeutet.

Die Reste $R_1$ und $R_2$, $R_1$ und $R_3$, $R_2$ und $R_7$ oder $R_3$ und $R_7$ können dabei zusammen eine Alkyl- oder Alkylenbrücke wie -$CH_2$-$CH_2$-, -$CH_2$-$CH(CH_3)$-, $CH_2$-$CH_2$-$CH_2$-, -$CH_2$-$CH(CH_3)$-$CH_2$- oder -$CH_2$-$C(CH_3)_2$-$CH_2$- bilden.

Besonders bevorzugt sind dabei Verbindungen in denen $R_1$, $R_2$, $R_3$ und $R_7$ unabhängig voneinander unsubstituiertes $C_1$-$C_4$-Alkyl wie Methyl oder Ethyl; $R_3$ Wasserstoff oder unsubstituiertes $C_1$-$C_4$-Alkyl wie Methyl, Ethyl, n-Propyl, i-Propyl, Butyl; bedeutet und n gleich 0 ist.

Der für die erfindungsgemässen Verbindungen verwendete Rest A kann dabei in bekannter Weise als Additionsprodukt von Ethylenoxid, Propylenoxid oder einer Mischung von beiden mit

a) einem gesättigten oder ungesättigten Fettalkohol mit 8 bis 26 C-Atomen, oder

b) einem Alkylphenol mit 4 bis 12 C-Atomen im Alkylrest, oder

c) einem Hydroxybiphenyl, oder

d) einem gesättigten oder ungesättigten Fettamin mit 8 bis 26 C-Atomen, oder

e) einer gesättigten oder ungesättigten Fettsäure mit 8 bis 26 C-Atomen, oder

f) einem gesättigten oder ungesättigten Fettsäureamid mit 8 bis 26 C-Atomen im Fettsäure- und 1 bis 10 C-Atomen im Amidsubstituenten,

hergestellt werden.

Bevorzugt sind dabei Verbindungen, bei denen A aus der Umsetzung einer Fettsäure mit 8 bis 18 C-Atomen, eines Fettalkohol mit 8 bis 18 C-Atomen oder eines Alkylphenols mit 6 bis 12 C-Atomen im

Alkylrest und Ethylenoxid oder Propylenoxid hervorgeht.

Beispielsweise aus der Additionsreaktion einer

a) gesättigten oder ungesättigten Fettsäure, wie Öl-, Linol-, Linolen-, Caprin-, Laurin-, Myristin-, Palmitin-, Stearin-, Kokosnuß- oder Palmkernsäure,

b) eines gesättigten oder ungesättigten Fettalkohols wie Nonyl-, Decyl-, Undecyl-, Lauryl-, Tridecyl-, Myristyl- Cetyl-, Oleyl-, Dodecenyl- oder Hexadecenylalkohol oder

c) eines Alkylphenoles mit 4 bis 12 C-Atomen im Alkylrest wie Nonylphenol

mit Ethylenoxid oder Propylenoxid, wobei auf 1 Mol der genannten Verbindungen vorzugsweise 4 bis 100 Mol Ethylenoxid oder eines Gemisches aus Ethylenoxid und Propylenoxid kommen. Besonders bevorzugt sind hierbei die Alkoholethoxylate. Es können aber auch Gemische dieser Umsetzungsprodukte untereinander verwendet werden. Diese Gemische erhält man durch Mischen einzelner Umsetzungsprodukte oder direkt durch Ethoxylierung eines Gemisches der den Umsetzungsprodukten zugrundeliegenden Verbindungen.

Die erfindungsgemässen Verbindungen erhält man durch eine Umesterung, wobei ein Orthoesters der Formel

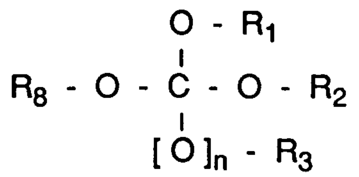

(2)

worin

$R_1$, $R_2$ und $R_3$ wie zuvor definiert sind; und

$R_8$ $C_1$-$C_{10}$-Alkyl;

bedeutet, mit einer Verbindung der Formel

$R_4$-X-($C_2$-$C_4$-Alkyl-O-)$_m$- $C_2$-$C_4$-Alkyl-OH (3)

worin $R_4$, X und m wie zuvor definiert sind, umgesetzt wird.

Die besten Resultate werden erhalten durch die Umsetzung von dem Orthoester der Formel (2) mit der Verbindung der Formel (3) im Verhältnis von 0.7 bis 5.0 Mol des Orthoesters pro Mol der Verbindung der Formel (3), insbesondere bei einem Verhältnis von 1:1.0 bis 4.0.

Die Reaktionsbedingungen werden dabei bevorzugt so gewählt, dass nur Monoester entstehen. Im Falle, dass man das Molverhältnis von Verbindung der Formel (3) zu Orthoester unter 1:3.0 wählt, werden Estergemische erhalten, die einen grösseren Anteil von Di- oder Triester enthalten.

Die Umsetzung findet dabei in Gegenwart eines aus der Literatur bekannten Katalysators für Umesterungen, bevorzugt in Gegenwart einer Säure oder eines Säureanhydrides statt.

Als Orthoester kommen hierbei besonders die Orthoester der Kohlensäure wie der Orthokohlensäuremethylester und der Orthokohlensäureethylester sowie Verbindungen wie Orthoameisensäuremethylester, Orthoameisensäureethylester, Orthoessigsäuremethylester, Orthoessigsäureethylester in Betracht.

Eine weitere Möglichkeit zur Herstellung der erfindungsgemässen Verbindungen besteht darin, dass man die Alkoholate der Formel

$R_4$-X-($C_2$-$C_4$-Alkyl-O-)$_m$-$C_2$-$C_4$-Alkyl-O-Na (K/Li)

mit z.B.$CHCl_3$ umsetzt.

Die erfindungsgemässen Verbindung werden bevorzugt ab einer Konzentration von 0,1 g/l eingesetzt.

Die Verbindungen können als Tenside, Dispergiermittel oder Detergentien eingesetzt werden. Sie sind in allen Bereichen, in denen eine geringe Schaumentwicklung erwünscht ist anwendbar, so z.B. zum Entschäumen von Wasser in der Enzymproduktion, als Zusatz zu Spülmitteln z.B. bei der Flasehenreinigung, zu Geschirrspülmitteln und im Haushalt, als Zusatz zu Waschmitteln oder als Textilhilfsmittel, z.B. beim Färben von Textilmaterialien.

Das erfindungsgemässe schaumarme Netzmittel ist dadurch gekennzeichnet, dass es:

a) 1 bis 100 Gew.% mindestens einer erfindungsgemässen Verbindung der Formel (1);

b) 0 bis 30 Gew.% mindestens eines Hydrotropiermittels;

c) 0 bis 10 Gew.% eines üblichen Hilfsmittels; und

d) ad 100 Gew.% Wasser, enthält.

Als Komponente (b) der erfindungsgemässen Zusammensetzung kommen folgende Hydrotropiermittel in Betracht:

i) Einwertige $C_4$ bis $C_{18}$ aliphatische und monocyclische Alkohole, wie $C_2$-$C_8$-Alkanole, $C_2$-$C_{18}$-Alkenole und Terpenalkohole, z.B. Propanol, Isopropanol, Hexanol, cis-3-Hexen-1-ol, trans-2-Hexen-1-ol, 1-Octen-3-ol, Heptanol, Octanol, trans-2-cis-6-Nonadien-1-ol, Decanol, Linanool, Geraniol, Dihydroterpinol, Myrcenol, Nopol und Terpineol;

ii) Aromatische Alkohole der Formel:

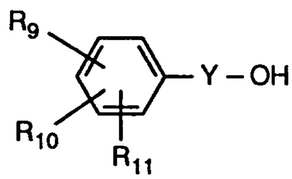

(4)

worin Y

$$-(CH_2)_{1-6}\ ,$$

-CH = CH-$CH_2$- oder

$$-O-(CH_2)_{2-6}$$

und $R_9$, $R_{10}$ und $R_{11}$, unabhängig voneinander, Wasserstoff, Hydroxy, Halogen oder $C_1$-$C_6$-Alkoxy bedeuten, wie z.B. Benzylalkohol, 2,4-Dichlorobenzylalkohol, Phenylethanol, Phenoxyethanol, 1-Phenoxy-2-propanol (Phenoxy-isopropanol) und Zimtalkohol; und

iii) Sulfonate von Terpenoiden oder ein- oder zweikernigen aromatischen Verbindungen, z.B. die Sulfonate des Camphers, Toluols, Xylols, Cumols und Naphthols.

Besonders bevorzugte erfindungsgemässe Hydrotropiermittel der Komponente (b) sind Alkylsulfate der Formel

$R_{12}O$-$SO_3Z$ (5)

worin

$R_{12}$ einen aliphatischen gesättigten, verzweigten oder geradkettigen Rest mit 4 bis 24 Kohlenstoffatomen und

Z Wasserstoff, Alkalimetall oder Ammonium, bedeutet.

Liegt das Alkylsulfat als Salz vor, so kommen beispielsweise Natrium-, Kalium- oder Ammoniumsalze in Betracht. Das Natriumsalz ist bevorzugt.

Ganz besonders bevorzugte Hydrotropiermittel der Komponente (b) sind Alkylsulfate, bei denen der Substituent $R_{12}$ in Formel (5) den Kohlenwasserstoffrest eines aliphatischen gesättigten Monoalkohols mit 4 bis 24 Kohlenstoffatome bedeutet. Der Kohlenwasserstoffrest kann geradkettig oder verzweigt sein.

Als aliphatische gesättigte Monoalkohole kommen dabei natürliche Alkohole in Betracht, wie z.B. Lauryl-, Myristyl-, Cetyl-, Stearyl-, Arachnidyl- oder Behenylalkohol. Bevorzugt sind Verbindungen, bei denen sich der Substituent $R_{12}$ von verzweigten aliphatischen synthetischen Alkoholen mit 4 bis 12, insbesondere 4 bis 8 Kohlenstoffatomen ableitet, z.B. Isobutylalkohol, sek.Butanol, tert.Butanol, Isoamylalkohol, 2-Ethylbutanol, 2-Methylpentanol, 5-Methylheptan-3-ol, 2-Ethylhexanol, 1,1,3,3-Tetramethylbutanol, Octan-2-ol, Isononylalkohol, Trimethylhexanol, Trimethylnonylalkohol, Decanol oder $C_9$-$C_{11}$-Oxoalkohol. Die Alkylsulfate können dabei bereits in Form ihrer Salze vorliegen und allein oder als (technisches) Gemisch untereinander in dem erfindungsgemässen Netzmittel eingesetzt werden.

Die Herstellung dieser Alkylsulfate erfolgt nach an sich bekannter Weise durch Umsetzung der entsprechenden Alkohole mit z.B. Schwefelsäure, Oleum, Chlorsulfonsäure oder Schwefeltrioxid.

Das erfindungsgemässe Netzmittel kann zusätzlich eine fakultative Komponente (c) enthalten.

Es kommen dafür unpolare organische Lösungsmittel in Betracht, deren Flammpunkt oberhalb von 65 °C liegt. Es können z.B. zyklische geradkettige oder insbesondere verzweigte Alkohole eingesetzt werden, wie z.B. Cyclohexanol, Methylcyclohexanol, n-Hexanol, 2-Ethylhexanol-1, Isooctylalkohol, Isononylalkohol und besonders Trimethylhexanol-3,5,5. Ferner können als unpolare organische Lösungsmittel Ester eingesetzt werden, wie zum Beispiel Tributylcitrat oder Tributylphosphat.

Die neuen Netzmittel können durch einfaches Verrühren der genannten Komponenten (a), (b) und gegebenenfalls (c) hergestellt werden.

Die Herstellung erfolgt vorzugsweise dadurch, dass man die Komponenten (a), (b) und gegebenenfalls (c) unter Rühren mischt und deionisiertes Wasser hinzugibt, bis eine homogene Lösung vorliegt.

Bevorzugte erfindungsgemässe Netzmittel enthalten insbesondere mit Vorteil, bezogen auf das gesamte Gemisch,
5 bis 98 Gew.% der Komponente (a),
2 bis 20 Gew.% der Komponente (b),
0 bis 4 Gew.% der Komponente (c) und
ad 100 % Wasser.

Die neuen Netzmittel stellen wässrige, in der Anwendung gering schäumende Formulierungen dar, die sich durch einen Trübungspunkt auszeichnen, der über 40 °C liegt und die bis 40 °C lagerstabil sind.

Sie finden Verwendung als Netzmittel in der Textilbehandlung, insbesondere in der Vorbehandlung, wie beispielsweise in der Langflottenbleiche oder in der Chlor- und Peroxid-Heissbleiche.

Gegenstand der vorliegenden Erfindung ist demnach auch ein Verfahren zum Netzen von Fasermaterialien. Das Verfahren ist dadurch gekennzeichnet, dass man diese Materialien in Gegenwart eines Netzmittels behandelt, das

(a) 10 bis 80 Gew.% mindestens einer Verbindung der allgemeinen Formel (1);
(b) 1 bis 10 Gew.% eines Hydrotropiermittels
(c) 0 bis 4 Gew.% eines unpolaren Lösungsmittels und ad 100 % Wasser enthält.

Die Einsatzmengen, in denen das erfindungsgemässe Netzmittel den Behandlungsflotten zugesetzt wird, betragen zwischen 0,1 und 10, vorzugsweise 0,5 und 5 g pro Liter Behandlungsflotte. Die Flotte kann noch weitere Zusätze enthalten, z.B. Entschlichtungsmittel, Farbstoffe, optische Aufheller, Kunstharze und Alkalien wie Natriumhydroxid.

Als Fasermaterialien kommen in Betracht:
Cellulose, insbesondere unvorbehandelte natürliche Cellulose wie z.B. Hanf, Leinen, Jute, Zellwolle, Viskose, Azetatreyon, native Cellulosefaser und besonders Rohbaumwolle, Wolle, Polyamid-, Polyacrylnitril- oder Polyesterfasermaterialien sowie Fasermischungen, z.B. solche aus Polyacrylnitril/Baumwolle oder Polyester/Baumwolle.

Die nachfolgenden Beispiele erläutern die Erfindung.

Beispiel 1: In einem Reaktionskolben werden 306 g eines Anlagerungsproduktes von 8 Mol Ethylenoxid an 1 Mol Decylalkohol bei Raumtemperatur vorgelegt, 2 g p-Toluolsulfonsäureanhydrid und 267 g Triethylorthoformiat eingerührt und unter verminderten Druck (700 mBar) bei 135 °C während 15 Stunden gerührt. In diesem Zeitraum destillieren 30 g Ethylalkohol aus dem Reaktionsgemisch ab. Die Temperatur wird anschliessend auf 90 °C gesenkt und das Vakuum schrittweise auf 30 mBar erhöht. Nach weiteren 3 Stunden wird das überschüssige Triethylformiat entfernt und man erhält 368 g Produkt (100 % der Theorie in Bezug auf Monoester).

Beispiel 2: Wie in Beispiel 1 beschrieben, werden 138 g des Anlagerungsproduktes vom 10 Mol Ethylenoxid an 1 Mol 4-Nonylphenol mit 90,7 g Triethylorthoformiat umgesetzt. Die Ausbeute an isolierten Produkt beträgt 168,3 g (105 % der Theorie bezogen auf den Monoester).

Das Produkt wird für die Applikation zu einem Mittel formuliert, das 15 % Wasser enthält.

Beispiel 3 : In einem Reaktionskolben werden 92,2 g eines Anlagerungsproduktes von 7 Mol Ethylenoxid an 1 Mol Decylalkohol bei Raumtemperatur vorgelegt, 0,7 g p-Toluolsulfonsäureanhydrid und 64,9 g Trimethylorthoformiat eingerührt. Die Reaktionsmasse wird während 4 Stunden auf eine Temperatur von 120-125 °C erhitzt und 3 Stunden bei dieser Temperatur gerührt. In diesem Zeitraum destillieren 13 g Destillat aus dem Reaktionsgemisch ab. Nach Einengen des Kolbeninhaltes im Vakuum erhält man 105 g Endprodukt (98 % der Theorie in Bezug auf Monoester).

Das Produkt wird für die Applikation als entsprechendes Mittel mit 15 % Wasser formuliert.

Beispiele 4 bis 10 : Setzt man, anstelle des Anlagerungsproduktes gemäss Beispiel 1, im gleichen Molverhältnis die folgenden Ethoxylate gemäss Tabelle 1 mit Triethylorthoformiat um, so erhält man ebenfalls schaumarme Tenside mit gutem Netzverhalten.

Tabelle 1

| Anlagerungsprodukt von n Mol Ethylenoxid an 1 Mol Fettalkohol | | |
|---|---|---|
| Beispiel | Fettalkohol | Ethylenoxid |
| 4 | Mischung aus $C_9$ bis $C_{11}$ Fettalkohol | 7 Mol |
| 5 | Mischung aus $C_9$ bis $C_{11}$ Fettalkohol | 8 Mol |
| 6 | Mischung aus $C_9$ bis $C_{11}$ Fettalkohol | 11 Mol |
| 7 | iso-$C_{13}$ Fettalkohol | 11 Mol |
| 8 | iso-$C_{13}$ Fettalkohol | 8 Mol |
| 9 | iso-$C_{13}$ Fettalkohol | 10 Mol |
| 10 | iso-$C_{13}$ Fettalkohol | 12 Mol |

Beispiel 11: Bei dem Produkt aus Beispiel 1 ermittelt man das Netzvermögen in Anlehnung an DIN 53901. Dazu wird die Konzentration bestimmt, bei der eine Rohbaumwoll-Rondelle in einer Zeit von 25 Sekunden versinkt Diese Konzentration beträgt bei dem Produkt aus Beispiel 1 0,9 g/l.

Zur Bestimmung des Schaumwertes gemäss DIN 53906 (Lochscheiben-Schlagmethode) wird das Schaumvolumen von 200 mi einer Lösung die 2 g/l des Produktes aus Beispiel 1 enthält gemessen. Das gemessene Schaumvolumen beträgt, sofort nach 60 Schlägen gemessen, 0 ml.

Beispiel 12 : Für das Produkt aus Beispiel 2 wird das Netzvermögen und der Schaumwert, wie in Beispiel 11 beschrieben, gemessen.

| | |
|---|---|
| Netzvermögen : | 1,8 g/l |
| Schaumwert : | 10 ml. |

Beispiel 13 : Für das Produkt aus Beispiel 3 wird das Netzvermögen und der Schaumwert, wie in Beispiel 11 beschrieben, gemessen.

| | |
|---|---|
| Netzvermögen : | 1,2 g/l |
| Schaumwert : | 10 ml. |

Beispiele 14 bis 20: Analog Beispiel 1 werden die in nachfolgender Tabelle aufgeführten Mengen an R-OH mit Orthoestern zu Endprodukten umgesetzt:

Tabelle 2:

| Beispiel Nr. | Alkohol | Menge (g) | Katalysator PTSA (g) | Orthoester Menge | (g) | Ausbeute in (g) | Netzen | Schaumwert (ml) 2 g/l nach 60 Sec. |
|---|---|---|---|---|---|---|---|---|
| 14 | PEG-400 Monooleat | 49.8 | 0.27 | TEOF | 34 | 60.9 | gut | 30 |
| 15 | Kokosfettamin + 15 Aeto | 65.7 | 0.27 | TEOF | 68 | 89.1 | gut | 0 |
| 16 | Kokosfettamin + 5 Aeto | 32.6 | 0.27 | TEOF | 68 | 50.5 | mässig | 0 |
| 17 | Kokosfettamin + 15 Aeto quat. mit Methylchlorid | 69.5 | 0.27 | TEOF | 68 | 93.7 | gut | 0 |
| 18 | Kokosfettsäure-monoäthanolamid + 8 Aeto | 45.1 | 0.27 | TEOF | 68 | 60.3 | s. gut | 10 |
| 19 | Sorbitmonolaurat + 4 Aeto | 16.2 | 0.54 | TEOF | 68 | 22.8 | mässig | 0 |
| 20 | Decanol + 12 Aeto | 130.3 | 0.70 | TEOAc | 99 | 165.8 | s. gut | 5 |

PTSA = p-Toluolsäureanhydrid
TEOF = Triäthylorthoformiat
TEOAc = Triäthylorthoacetat
Aeto = Ethylenoxid

Beispiele 21 bis 23: Setzt man, anstelle des Anlagerungsproduktes gemäss Beispiel 1, die folgenden Alkohole gemäss Tabelle 3 mit Triethylorthoformiat (TEOF), in angegebenem Molverhältnis um, so erhält man ebenfalls schaumarme Tenside mit gutem Netzverhalten:

Tabelle 3

| Beispiel Nr. | Alkohol | Netzvermögen g/l für 25 Sek. | Schaumvolumen (ml) 2 g/l (sofort/nach 1 min.) |
|---|---|---|---|
| 21 | Decanol + 9 Aeto | 1.2 | 10 / 0 |
| 22 | Decanol + 10 Aeto | 1.16 | 20 / 0 |
| 23 | Decanol + 12 Aeto | 1.4 | 20 / 0 |

Anlagerungsprodukte gemäss Beispiele 22 und 23 werden mit 0.25 % Na-methylat formuliert.

Beispiel 24: Analog Beispiel 3 werden 215,8 g eines Anlagerungsproduktes von 9 Mol Aethylenoxid an 1-Decanol mit 129,8 g Trimethylorthoformiat unter Zusatz von 1,4 g p-Toluolsäureanhydrid umgesetzt. Man erhält 239,0 g eines Tensidgemisches mit einem niedrigen Anteil an Monoester. Das erhaltene Produkt ist ein sehr wirksames, schaumfreies Netzmittel.

Beispiel 25: Formulierung als schaumarmes Netzmittel.
Die folgenden Komponenten werden bei Raumtemperatur unter Rühren zusammengemischt. Es entsteht eine farblose klare Formulierung mit einem Trübungspunkt von 74 °C.

Zusammensetzung:
25 % Decanol + 9 Aeto mit TEOF umgesetzt
4 % Texapon EHS (40%-iges 2-Ethylhexylsulfat)
3 % 3,5,5-Trimethylhexanol
63 % Wasser.

Beispiel 26: Prüfung von der Formulierung des Beispiels 25 in der Langflotten-Bleiche.

Ein Roh-Baumwolltricot wird im ®Ahiba-Färbeapparat in einem Bleichbad gebleicht, welches im Liter
1 g Formulierung des Beispiels 25
0,5 g eines handelsüblichen Bleichstabilisators
1,5 g NaOH (100 %) und
4 ml 35 % $H_2O_2$
enthält.

Das Bleichbad wird innerhalb von 20 Min. auf 90 °C aufgeheizt und anschliessend weitere 30 Min. bei dieser Temperatur gehalten. Während der ganzen Bleichdauer tritt keine störende Schaumbildung auf. Das Schaumverhalten des Bleichbades wird zusätzlich bei Raumtemperatur vor und nach der Bleiche gemäss DIN 53902 geprüft.
Schaumhöhe vor der Bleiche: 5 ml
Schaumhöhe nach der Bleiche: 0 ml.

## Patentansprüche

**1.** Verbindung der Formel (1)

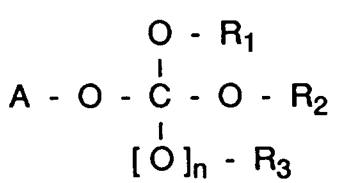

(1)

worin

| | |
|---|---|
| $R_1$ und $R_2$ | unabhängig voneinander unsubstituiertes oder substituiertes $C_1$-$C_{10}$-Alkyl, $C_2$-$C_{10}$-Alkenyl oder $R_1$ gleich A; |
| n | 0 oder 1; |
| $R_3$ | Wasserstoff, unsubstituiertes oder substituiertes $C_1$-$C_{10}$-Alkyl, oder $C_2$-$C_{10}$-Alkenyl; oder |
| $R_1$ und $R_2$ | gemeinsam eine geradkettige oder verzweigte $C_2$-$C_5$-Alkylenbrücke; und die Gesamtzahl der C-Atome in $R_1$, $R_2$ und $R_3$ zusammen nicht mehr als 10 beträgt, wobei die C-Atome für $R_1$ gleich A nicht berücksichtigt werden; |
| A | den Rest $R_4$-X-($C_2$-$C_4$-Alkyl-O-$)_m$-$C_2$-$C_4$-Alkyl-; |

| | |
|---|---|
| $R_4$ | unsubstituiertes oder substituiertes $C_8$-$C_{26}$-Alkyl oder $C_8$-$C_{26}$-Alkenyl; im Phenylrest substituiertes Phenyl, Biphenyl oder Phenyl-$C_1$-$C_{13}$-Alkyl; |
| X | |

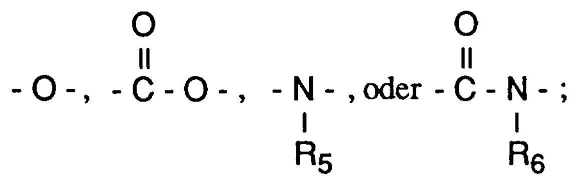

| | |
|---|---|
| $R_5$ und $R_6$ | unabhängig voneinander Wasserstoff, unsubstituiertes oder substituiertes $C_1$-$C_8$-Alkyl, -($C_2$-$C_4$-Alkyl-O-)$_o$- $C_2$-$C_4$-Alkyl-OH, oder |

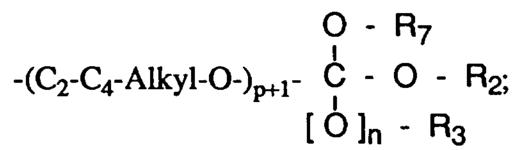

| | |
|---|---|
| $R_7$ | unsubstituiertes oder substituiertes $C_1$-$C_{10}$-Alkyl, oder $C_2$-$C_{10}$-Alkenyl; und |
| m, o und p | unabhängig voneinander 0 bis 100 bedeutet. |

**2.** Verbindungen gemäss Anspruch 1, worin

| | |
|---|---|
| $R_1$ und $R_2$ | unabhängig voneinander unsubstituiertes oder substituiertes $C_1$-$C_{10}$-Alkyl; oder $C_2$-$C_{10}$-Alkenyl; |
| n | 0 oder 1; |
| $R_3$ | Wasserstoff; unsubstituiertes oder substituiertes $C_1$-$C_{10}$-Alkyl; oder $C_2$-$C_{10}$-Alkenyl; oder |
| $R_1$ und $R_2$ | gemeinsam eine geradkettige oder verzweigte $C_2$-$C_5$-Alkylenbrücke; |

bedeutet und die Gesamtzähl der C-Atome in $R_1$, $R_2$ und $R_3$ zusammen nicht mehr als 10 beträgt.

**3.** Verbindungen gemäss Anspruch 1 oder 2, worin $R_4$ substituiertes $C_8$-$C_{18}$-Alkyl oder $C_6$-$C_{12}$-Alkylphenyl bedeutet.

**4.** Verbindungen gemäss Anspruch 1 oder 2, worin $R_4$ unsubstituiertes $C_8$-$C_{18}$-Alkyl oder $C_6$-$C_{12}$-Alkylphenyl bedeutet.

**5.** Verbindung gemäss Anspruch 1 bis 4, worin X

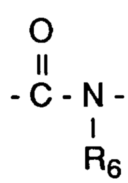

bedeutet.

**6.** Verbindung gemäss Anspruch 1 bis 4, worin X -O- bedeutet.

**7.** Verbindung gemäss einem der Ansprüche 1 bis 6, worin $R_1$, $R_2$ und $R_7$ unabhängig voneinander unsubstituiertes $C_1$-$C_4$-Alkyl oder $C_2$-$C_4$-Alkenyl; $R_3$ Wasserstoff oder unsubstituiertes $C_1$-$C_4$-Alkyl oder $C_2$-$C_4$-Alkenyl; oder $R_1$ und $R_2$, $R_1$ und $R_3$, $R_7$ und $R_2$ oder $R_7$ und $R_3$ gemeinsam eine $C_2$-$C_4$-Alkylenbrücke bedeutet.

**8.** Verbindung gemäss einem der Ansprüche 1 bis 7, worin $R_1$ , $R_2$ und $R_7$ unabhängig voneinander unsubstituiertes $C_1$-$C_4$-Alkyl; $R_3$ Wasserstoff oder unsubstitu-

iertes $C_1$-$C_4$-Alkyl; und n 0 bedeutet.

**9.** Verbindung gemäss einem der Ansprüche 1 bis 8, worin
$R_1$, $R_2$ und $R_7$ unabhängig voneinander Methyl oder Ethyl; $R_3$ Wasserstoff; und n 0 bedeutet.

**10.** Verbindung gemäss Anspruch 1, worin $R_1$, $R_2$ und $R_7$ unabhängig voneinander Methyl oder Ethyl; $R_3$ Wasserstoff;
$R_4$ $C_8$-$C_{13}$-Alkyl oder $C_6$-$C_{12}$-Alkylphenyl;
X -O- ; und
n 0 bedeutet.

**11.** Verfahren zur Herstellung einer Verbindung gemäss einem der Ansprüche 1 bis 10, gekennzeichnet durch die Umsetzung eines Orthoesters der Formel

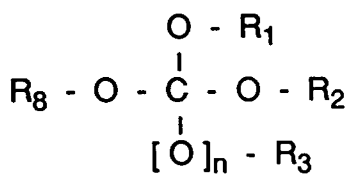 (2)

worin
$R_1$, $R_2$, $R_3$ und n wie in Anspruch 1 definiert sind; und
$R_8$ $C_1$-$C_{10}$-Alkyl;
bedeutet, mit einer Verbindung der Formel

$R_4$-X-($C_2$-$C_4$-Alkyl-O-$)_m$- $C_2$-$C_4$-Alkyl-OH (3)

worin $R_4$, X und m wie in Anspruch 1 definiert sind.

**12.** Verfahren gemäss Anspruch 11, dadurch gekennzeichnet, dass die Umsetzung im Verhältnis von 1:1.0 bis 4.0 Mol des Orthoesters pro Mol der Verbindung der Formel (3), stattfindet.

**13.** Verfahren gemäss Anspruch 11 oder 12, dadurch gekennzeichnet, dass die Umsetzung in Gegenwart einer Säure oder eines Säureanhydrides stattfindet.

**14.** Verfahren gemäss einem der Ansprüche 11 bis 13, dadurch gekennzeichnet, dass man das Anlagerungsprodukt von Ethylenoxid an einen Fettalkohol oder an Nonylphenol in Gegenwart von p-Toluolsulfonsäureanhydrid mit Triethylorthoformiat oder Trimethylorthoformiat bei erhöhter Temperatur umsetzt und das Endprodukt durch Destillation reinigt.

**15.** Verwendung der Verbindungen der Ansprüche 1 bis 10 als Tenside, Dispergiermittel oder Detergentien.

**16.** Schaumarmes Netzmittel, dadurch gekennzeichnet, dass es
a) 1 bis 100 Gew.% mindestens einer Verbindung der Formel (1) gemäss einem der Ansprüche 1 bis 10;
b) 0 bis 30 Gew.% mindestens eines Hydrotropiermittels;
c) 0 bis 10 Gew.% eines üblichen Hilfsmittel; und
d) ad 100 Gew.% Wasser,
enthält.

**17.** Schaumarmes Netzmittel gemäss Anspruch 16, dadurch gekennzeichnet, dass das Hydrotropiermittel der Komponente (b) der Formel

$R_{12}O$-$SO_3Z$ (5)

entspricht, worin

$R_{12}$ einen aliphatischen gesättigten, verzweigten oder geradkettigen Rest mit 4 bis 24 Kohlenstoffatomen und
Z Wasserstoff, Alkalimetall oder Ammonium, bedeutet.

**18.** Schaumarmes Netzmittel gemäss Anspruch 17, dadurch gekennzeichnet, dass der Substituent $R_{12}$ den Kohlenwasserstoffrest eines aliphatischen gesättigen Monoalkohols mit 4 bis 24 Kohlenstoffatome bedeutet.

**19.** Schaumarmes Netzmittel gemäss Anspruch 17 oder 18, dadurch gekennzeichnet, dass die fakultative Komponente (c) ein unpolares organisches Lösungsmittel bedeutet, dessen Flammpunkt oberhalb von 65C° liegt.

**20.** Schaumarmes Netzmittel gemäss Anspruch 19, dadurch gekennzeichnet, dass das Lösungsmittel Trimethylhexanol-3,5,5 bedeutet.

**21.** Schaumarmes Netzmittel gemäss einem der Ansprüche 16 bis 20, dadurch gekennzeichnet, dass es
5 bis 98 Gew.% der Komponente a);
2 bis 20 Gew.% der Komponente b);
0 bis 4 Gew.% der Komponente c); und
ad 100 Gew.% Wasser,
enthält.

## Claims

**1.** A compound of the formula (1)

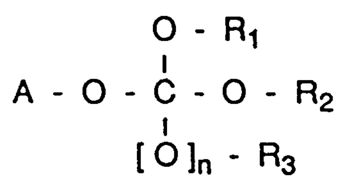
(1)

in which
$R_1$ and $R_2$ are, independently of one another, unsubstituted or substituted $C_1$-$C_{10}$alkyl, $C_2$-$C_{10}$alkenyl or $R_1$ is identical to A;
n is 0 or 1;
$R_3$ is hydrogen, unsubstituted or substituted $C_1$-$C_{10}$alkyl, or
$C_2$-$C_{10}$alkenyl; or $R_1$ and $R_2$ together are a straight-chain or branched $C_2$-$C_5$alkylene bridge; and the total number of C atoms in $R_1$, $R_2$ and $R_3$ together is not more than 10, not taking the C atoms for $R_1$ identical to A into account;
A is the radical $R_4$-X-($C_2$-$C_4$alkyl-O)$_m$-$C_2$-$C_4$alkyl-;
$R_4$ is unsubstituted or substituted $C_8$-$C_{26}$alkyl or $C_8$-$C_{26}$alkenyl; phenyl, biphenyl or phenyl-$C_1$-$C_{13}$alkyl, each of which is substituted in the phenyl radical; X is

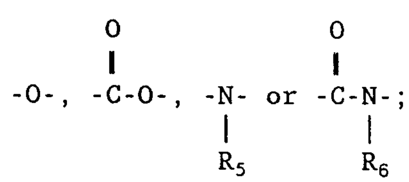

$R_5$ and $R_6$ are, independently of one another, hydrogen, unsubstituted or substituted $C_1$-$C_8$alkyl, -($C_2$-$C_4$alkyl-O)$_o$- $C_2$-$C_4$alkyl-OH, or

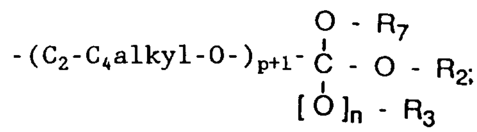

$R_7$ is unsubstituted or substituted $C_1$-$C_{10}$alkyl or $C_2$-$C_{10}$alkenyl; and
m, o and p are, independently of one another, 0 to 100.

**2.** A compound according to claim 1, in which
$R_1$ and $R_2$ are, independently of one another, unsubstituted or substituted $C_1$-$C_{10}$alkyl; or $C_2$-$C_{10}$alkenyl;
n is 0 or 1;
$R_3$ is hydrogen; unsubstituted or substituted $C_1$-$C_{10}$alkyl; or
$C_2$-$C_{10}$alkenyl; or
$R_1$ and $R_2$ together are a straight-chain or branched $C_2$-$C_5$alkylene bridge; and the total number of C atoms in $R_1$, $R_2$ and $R_3$ together is not more than 10.

**3.** A compound according to claim 1 or 2, in which $R_4$ is substituted $C_8$-$C_{18}$alkyl or $C_6$-$C_{12}$alkylphenyl.

**4.** A compound according to claim 1 or 2, in which $R_4$ is unsubstituted $C_8$-$C_{18}$alkyl or $C_6$-$C_{12}$alkylphenyl.

**5.** A compound according to claim 1 to 4, in which X is

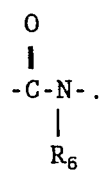

**6.** A compound according to claim 1 to 4, in which X is -O-.

**7.** A compound according to any one of claims 1 to 6, in which $R_1$, $R_2$ and $R_7$ are, independently of one another, unsubstituted $C_1$-$C_4$alkyl or $C_2$-$C_4$alkenyl;
$R_3$ is hydrogen or unsubstituted $C_1$-$C_4$alkyl or $C_2$-$C_4$alkenyl; or
$R_1$ and $R_2$, $R_1$ and $R_3$, $R_7$ and $R_2$ or $R_7$ and $R_3$ together are a $C_2$-$C_4$alkylene bridge.

**8.** A compound according to any one of claims 1 to 7, in which
$R_1$, $R_2$ and $R_7$ are, independently of one another, unsubstituted $C_1$-$C_4$alkyl;
$R_3$ is hydrogen or unsubstituted $C_1$-$C_4$alkyl; and
n is 0.

**9.** A compound according to any one of claims 1 to 8, in which
$R_1$, $R_2$ and $R_7$ are, independently of one another, methyl or ethyl;
$R_3$ is hydrogen; and
n is 0.

**10.** A compound according to claim 1, in which
$R_1$, $R_2$ and $R_7$ are, independently of one another, methyl or ethyl;
$R_3$ is hydrogen;
$R_4$ is $C_8$-$C_{13}$alkyl or $C_6$-$C_{12}$alkylphenyl;
X is -O-; and
n is 0.

**11.** A process for the preparation of a compound according to any one of claims 1 to 10, wherein an orthoester of the formula

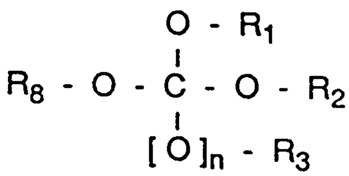

(2)

in which
$R_1$, $R_2$, $R_3$ and n are as defined in claim 1; and
$R_8$ is $C_1$-$C_{10}$alkyl;
is reacted with a compound of the formula

$R_4$-X-($C_2$-$C_4$alkyl-O-)$_m$-$C_2$-$C_4$alkyl-OH (3)

in which $R_4$, X and m are as defined in claim 1.

**12.** A process according to claim 11, wherein the reaction place in the ratio of 1:1.0 to 4.0 mol of the orthoester per mol of the compound of the formula (3).

**13.** A process according to claim 11 or 12, wherein the reaction takes place in the presence of an acid or of an acid anhydride.

**14.** A process according to any one of claims 11 to 13, wherein the addition product of ethylene oxide and a fatty alcohol or nonylphenol is reacted in the presence of p-toluenesulfonic anhydride with triethyl orthoformate or trimethyl orthoformate at elevated temperature, and the final product is purified by distillation.

**15.** The use of the compounds of claims 1 to 10 as surfactants, dispersants or detergents.

**16.** A low-foaming wetting agent, which comprises
a) 1 to 100% by weight of at least one compound of the formula (1) according to any one of claims 1 to 10;
b) 0 to 30% by weight of at least one hydrotropic agent;
c) 0 to 10% by weight of a conventional auxiliary; and
d) water to 100% by weight.

**17.** A low-foaming wetting agent according to claim 16, wherein the hydrotropic agent of component (b) corresponds to the formula

$R_{12}O-SO_3Z$ (5)

in which
$R_{12}$ is an aliphatic saturated, branched or straight-chain radical with 4 to 24 carbon atoms and
Z is hydrogen, alkali metal or ammonium.

**18.** A low-foaming wetting agent according to claim 17, wherein the substituent $R_{12}$ is the hydrocarbon radical of an aliphatic saturated monoalcohol with 4 to 24 carbon atoms.

**19.** A low-foaming wetting agent according to claim 17 or 18, wherein the optional component (c) is a non-polar organic solvent whose flash point is above 65 °C.

**20.** A low-foaming wetting agent according to claim 19, wherein the solvent is 3,5,5-trimethylhexanol.

**21.** A low-foaming wetting agent according to any one of claims 16 to 20, which comprises
5 to 98% by weight of component a);
2 to 20% by weight of component b);
0 to 4% by weight of component c); and
water to 100% by weight.

## Revendications

**1.** Composé de formule (1)

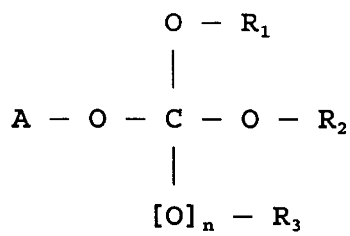

dans laquelle

- $R_1$ et $R_2$ représentent chacun, indépendamment l'un de l'autre, un groupe alkyle en $C_{1-10}$ ou alcényle en $C_{2-10}$, portant ou non des substituants, ou encore $R_1$ est identique à A,
- n vaut 0 ou 1,
- $R_3$ représente un atome d'hydrogène ou un groupe alkyle en $C_{1-10}$ ou alcényle en $C_{2-10}$, portant ou non des substituants,
  ou bien $R_1$ et $R_2$ représentent ensemble un groupe pontant alkylène en $C_{2-5}$, linéaire ou ramifié, le nombre total d'atomes de carbone dans $R_1$, $R_2$ et $R_3$ considérés ensemble ne valant pas plus de 10, mais les atomes de carbone de $R_1$ n'étant pas considérés dans cette somme quand $R_1$ est identique à A,
- A représente un reste $R_4$-X-[(alkyle en $C_{2-4}$)-O-]$_m$-(alkyle en $C_{2-4}$),
- $R_4$ représente un groupe alkyle en $C_{8-26}$ ou alcényle en $C_{8-26}$, portant ou non des substituants, ou un groupe phényle, biphényle ou phényl-(alkyle en $C_{1-13}$), dont les noyaux phényle portent des substituants,
- X représente -O-, -COO-, -N($R_5$)- ou -CO-N($R_6$)-,
- $R_5$ et $R_6$ représentent chacun, indépendamment l'un de l'autre, un atome d'hydrogène ou un groupe alkyle en $C_{1-8}$,
  -[(alkyle en $C_{2-4}$)-O-]$_o$-(alkyle en $C_{2-4}$)-OH ou

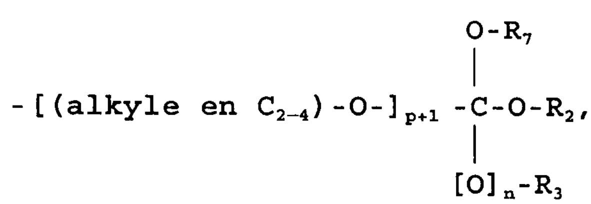

  portant ou non des substituants,
- $R_7$ représente un groupe alkyle en $C_{1-10}$ ou alcényle en $C_{2-10}$, portant ou non des substituants, et
- m, o et p valent chacun, indépendamment l'un de l'autre, de 0 à 100.

**2.** Composés conformes à la revendication 1, dans lesquels

- $R_1$ et $R_2$ représentent chacun, indépendamment l'un de l'autre, un groupe alkyle en $C_{1-10}$ portant ou non des substituants ou un groupe alcényle en $C_{2-10}$,
- n vaut 0 ou 1,
- $R_3$ représente un atome d'hydrogène, un groupe alkyle en $C_{1-10}$ portant ou non des substituants, ou un groupe alcényle en $C_{2-10}$,

ou $R_1$ et $R_2$ représentent ensemble un groupe pontant alkylène en $C_{2-5}$, linéaire ou ramifié,

le nombre total d'atomes de carbones dans $R_1$, $R_2$ et $R_3$ considérés ensemble ne dépassant pas 10.

**3.** Composés conformes à la revendication 1 ou 2, dans lesquels $R_4$ représente un groupe alkyle en $C_{8-18}$ substitué ou un groupe (alkyle en $C_{6-12}$)-phényle.

**4.** Composés conformes à la revendication 1 ou 2, dans lesquels $R_4$ représente un groupe alkyle en $C_{8-18}$ non substitué ou un groupe (alkyle en $C_{6-12}$)-phényle.

**5.** Composé conforme à l'une des revendications 1 à 4, dans lequel X représente -CO-N($R_6$)-.

**6.** Composé conforme à l'une des revendications 1 à 4, dans lequel X représente -O-.

**7.** Composé conforme à l'une des revendications 1 à 6, dans lequel $R_1$, $R_2$ et $R_7$ représentent chacun, indépendamment l'un de l'autre, un groupe alkyle en $C_{1-4}$ non substitué ou un groupe alcényle en $C_{2-4}$, $R_3$ représente un atome d'hydrogène ou un groupe alkyle en $C_{1-4}$ non substitué ou alcényle en $C_{2-4}$, ou bien $R_1$ et $R_2$, $R_1$ et $R_3$, $R_7$ et $R_2$, ou $R_7$ et $R_3$ forment ensemble un pont alkylène en $C_{2-4}$.

**8.** Composé conforme à l'une des revendications 1 à 7, dans lequel $R_1$, $R_2$ et $R_7$ représentent chacun, indépendamment l'un de l'autre, un groupe alkyle en $C_{1-4}$ non substitué, $R_3$ représente un atome d'hydrogène ou un groupe alkyle en $C_{1-4}$ non substitué, et n vaut 0.

**9.** Composé conforme à l'une des revendications 1 à 8, dans lequel $R_1$, $R_2$ et $R_7$ représentent chacun, indépendamment l'un de l'autre, un groupe méthyle ou éthyle, $R_3$ représente un atome d'hydrogène, et n vaut 0.

**10.** Composé conforme à la revendication 1, dans lequel $R_1$, $R_2$ et $R_7$ représentent chacun, indépendamment l'un de l'autre, un groupe méthyle ou éthyle, $R_3$ représente un atome d'hydrogène, $R_4$ représente un groupe alkyle en $C_{8-13}$ ou un groupe (alkyle en $C_{6-12}$)-phényle, X représente -O-, et n vaut 0.

**11.** Procédé de préparation d'un composé conforme à l'une des revendications 1 à 10, caractérisé en ce que l'on effectue la réaction d'un orthoester de formule

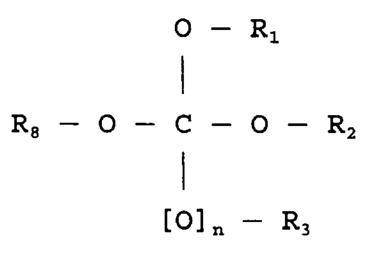

(2)

dans laquelle

$R_1$, $R_2$, $R_3$ et n ont les définitions indiquées dans la revendication 1, et $R_8$ représente un groupe alkyle en $C_{1-10}$,

avec un composé de formule

$R_4$-X-[(alkyle en $C_{2-4}$)-O-]$_m$-(alkyle en $C_{2-4}$)-OH (3)

dans laquelle $R_4$, X et m ont les définitions indiquées dans la revendication 1.

**12.** Procédé conforme à la revendication 11, caractérisé en ce qu'on réalise la réaction avec des proportions de 1,0 à 4,0 moles de l'orthoester par mole de composé de formule (3).

**13.** Procédé conforme à la revendication 11 ou 12, caractérisé en ce que la réaction a lieu en présence d'un acide ou d'un anhydride d'acide.

**14.** Procédé conforme à l'une des revendications 11 à 13, caractérisé en ce que l'on fait réagir de l'orthoformiate de triéthyle ou de l'orthoformiate de triméthyle et du produit d'addition d'oxyde d'éthylène sur un alcool gras ou sur du nonylphénol, en présence d'anhydride d'acide p-toluènesulfonique, à température élevée, et en ce que l'on purifie le produit final par distillation.

**15.** Utilisation de composés conforme à l'une des revendications 1 à 10, comme agents tensio-actifs, dispersants ou détergeants.

**16.** Agent mouillant peu moussant, caractérisé en ce qu'il contient

a) de 1 à 100 % en poids d'au moins un composé de formule (1) conforme à l'une des revendications 1 à 10,
b) de 0 à 30 % en poids d'au moins un agent d'hydrotropie,
c) de 0 à 10 % en poids d'un adjuvant habituel, et
d) le complément à 100 % en poids d'eau.

**17.** Agent mouillant peu moussant conforme à la revendication 16, caractérisé en ce que l'agent d'hydrotropie du composant (b) correspond à la formule

$R_{12}O\text{-}SO_3Z$

dans laquelle

$R_{12}$ représente un groupe aliphatique saturé, à chaîne droite ou ramifiée et comportant de 4 à 24 atomes de carbone, et

Z représente un atome d'hydrogène ou de métal alcalin ou un groupe ammonium.

**18.** Agent mouillant peu moussant conforme à la revendication 17, caractérisé en ce que le substituant $R_{12}$ représente le reste hydrocarboné d'un monoalcool saturé aliphatique comportant de 4 à 24 atomes de carbones.

**19.** Agent mouillant peu moussant conforme à la revendication 17 ou 18, caractérisé en ce que le composant facultatif (c) est un solvant organique non polaire dont le point d'inflammation se situe audessus de 65 °C.

**20.** Agent mouillant peu moussant conforme à la revendication 19, caractérisé en ce que le solvant est du 3,5,5-triméthylhexanol.

**21.** Agent mouillant peu moussant conforme à l'une des revendications 16 à 20, caractérisé en ce qu'il contient de 5 à 98 % en poids du composant (a), de 2 à 20 % en poids du composant (b) et de 0 à 4 % en poids du composant (c), le complément à 100 % en poids étant de l'eau.